# EUROPEAN PATENT APPLICATION

(11) **EP 1 486 900 A1**
(43) Date of publication of application: **15.12.2004**
(21) Application number: 03447149.0
(22) Date of filing: 12.06.2003
(51) Int. Cl.: G06F 19/00, A61C 1/08

(54) **Method and system for manufacturing a surgical guide**

(71) Applicant: Materialise, Naamloze Vennootschap, 3001 Heverlee (BE)
(72) Inventor: Swaelens, Bart Filip Jos, B-2580 Putte (BE); van Lierde, Carl, B-3000 Leuven (BE)
(74) Representative: Bird, William Edward

(57) **Abstract**

A method and apparatus are described for (i) combining a computer planning based on one or more medical images of the patient with geometrical information of the supporting surfaces in digital format (ii) processing these data in order to obtain a digital design of a custom guiding template that (iii) can be manufactured, for example, by means of CAM techniques. In order to be able to combine the detailed information of the supporting geometries mentioned above with the planning information, the digital description of the surfaces must be positioned to the corresponding surfaces present in the medical images identically in a suitable display space by means of computer registration.

## Description

### FIELD OF THE INVENTION

The present invention relates to a method for the design of supported guiding templates or precursors of supported guiding templates and optionally for the production of supported guiding templates or precursors of supported guiding templates based on patient specific information, in which this information has been brought into a digital format and is used for the design of the template or precursors of a template, as well as a computer based system for the design and/or production of the supported guiding template or a precursor of a template and software for use with the system.

### TECHNICAL BACKGROUND

A guiding template or jig for use in general or specific surgical procedures such as in dentistry may be defined as any object for placement on a unique and preferably stable position, specific for a patient and intended to be used for guiding a tool, an appliance or a medical or surgical device according to a prior determined position, direction and/or path and/or depth.

Typically a guiding template will consist of two pieces: (i) a structure providing a good or an optimal fit (i.e. the contact element) to the anatomy, usually a local anatomy of a patient and (ii) one or more guiding elements. Both pieces can be designed separately provided that correct integration of the two pieces is possible afterwards, i.e. before the surgical intervention. The production of a guiding template requires firstly a detailed, geometrical patient specific information about those surfaces usable for supporting the guide and secondly information about how the tools should be guided per-operatively. The first requirement is clarified below. The second, implies pre-operative planning of the intervention by the treating physician. Usually this is done in a dedicated software (such as SimPlant^{TM} as supplied by Materialise N.V., Leuven, Belgium) based on suitable medical images (of which CT, MRI, PET, ultrasound are examples), taking into account such factors as bone quality and proximity to nerve bundles/blood vessels or other anatomically sensitive objects.

A useful summary of medical imaging may be found in the book by P. Suetens, "Fundamentals of Medical imaging", Cambridge University Press, 2002. Image guided intervention (also called computer assisted intervention) is discussed in chapter 10. A synthetic mould or template can be manufactured, e.g. by stereolithography, that uniquely fits onto, around or into the relevant anatomy of the patient and contains precise openings to guide surgical instruments. (cf. WO9528688 Method for making a perfected medical model on the basis of digital image information of a part of the body, Materialise N.V.)

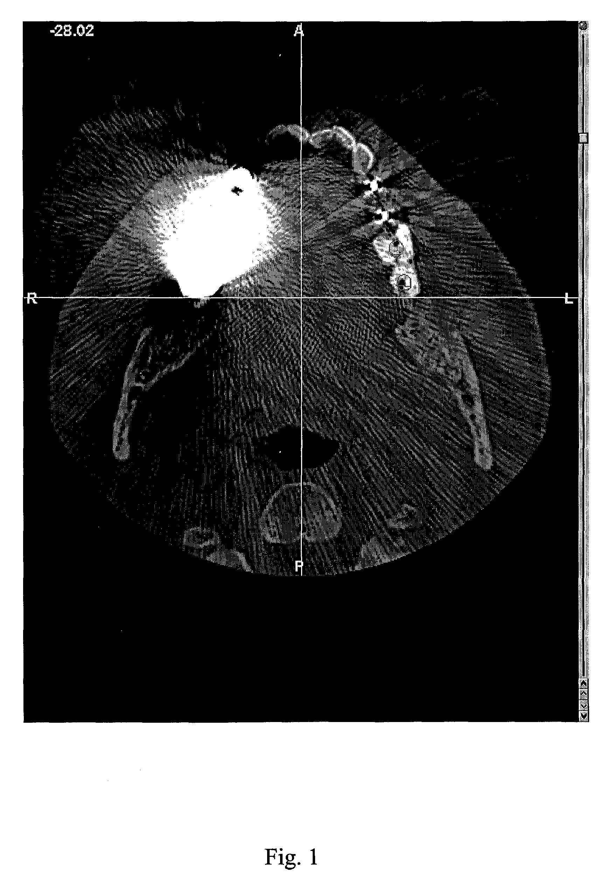

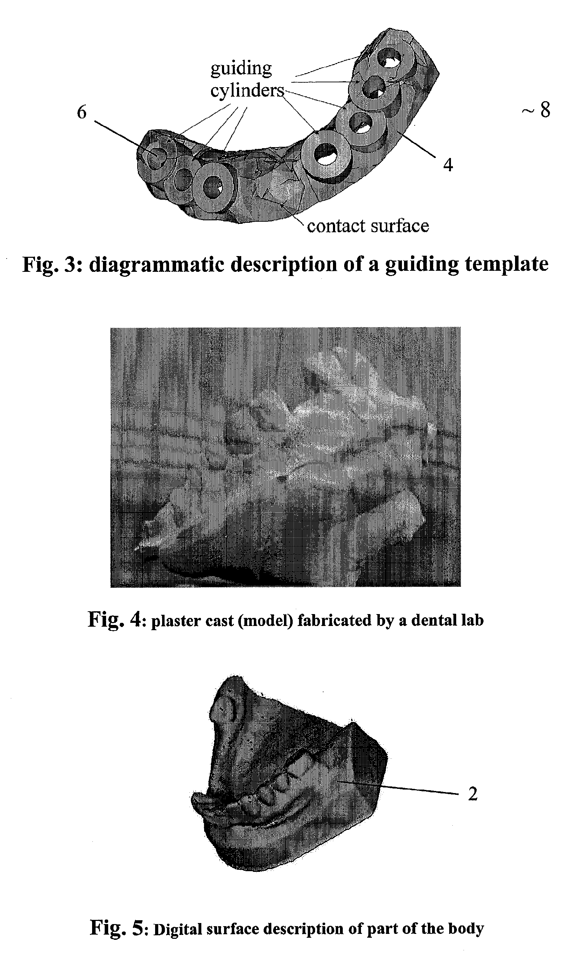

An important limitation of using medical imaging techniques to digitally capture patient related geometries is the presence of so called artifacts. Some types of such artifacts are shown in Fig. 5.1.6 of the above book and Fig. 1 attached. In CT image data these artifacts are often caused by metal objects present such as tooth fillings, implants, screws, braces, crowns, etc. These artifacts manifest themselves in sequential sections as scatter (i.e. discontinuous and irregular areas of high and low intensity gray values), compromising any attempt to delineate accurately the contours of the anatomical structures as well as masking some structures.

In addition, most volumetric imaging techniques have a limited resolution, rendering small details indiscriminatable. Consequently, some surface mismatching may occur when designing guiding templates solely based on these types of images. For templates which are to be supported on hard materials such as teeth or metal implants, a high degree of conformity of the template to the support is preferred, particularly when the surgical invasion goes deep. In such a case, small angular errors may result in significant positional errors at the end of the tool trajectory. One way of working is based on making an impression of the support structures. This impression can be converted to a model (typically plaster models are used) using casting techniques. According to known methods, the model can then be captured digitally by means of a mechanical or optical scanner, either as a point cloud or a surface description (triangular or other).

Alternative solutions are known in which the geometry is measured either directly on the patient or indirectly by scanning objects (other than models) containing detailed information about the geometrical characteristics of the anatomical support structures. An example, is the Cerec^{TM} system as described for instance in "Adhesive Metal-free Restorations", D. Dietschi and R. Spreafico, Quintessence Publishing, 1999, chapter 9. This involves an optical impression taken in the mouth of the patient. It is claimed that this optical detection method provides a resolution of 25 micron in all three axes. However, the type of intervention for which this system is designed only involves minor interventions with respect to teeth, e.g. overlays or inlays. Several different methods have been tried to record spatial coordinates of prepared teeth: laser holography, laser scanners, stereo photographs, mechanical digitizers. Independent of the technique used, the result should always be an accurate and detailed digital description of the surfaces.

Possible limitations inherent to the measuring/scanning equipment with respect to solving undercuts can be compensated by capturing the structure using a sufficient number of different viewing angles thus obtaining an entire 3 dimensional description.

A fundamental aspect of the design of the guiding templates entails prior identification of the orientations desired for guiding elements. For implantology this can be done based on information about the quantity and quality of the bone in dedicated computer software. To plan and simulate the intervention, preoperative images are imported into a computer workstation running 3D graphics software. These images are manipulated as 3D volumes. The result is a computer simulation of the intervention, which outputs a planning containing the information necessary for creating the guiding elements.

Another method according to the prior art uses a scan prosthesis fitted with radio-opaque fiducial markers made beforehand (WO9926540 Surgical template assembly and method for drilling and installing dental implants, M. Klein)

Computer tomography can be used to scan the patient with the scan prosthesis and a pre-operative simulation of the intervention is performed in a dedicated software. The relative positions of the planned implants are calculated with respect to the fiducial markers in the medical images. Afterwards the original scan prosthesis is modified by milling apertures along the axis of the planned implants. An important disadvantage of this system is that it is a circuitous way of working. The production of a guiding template requires a special scan prosthesis to be made beforehand. This prosthesis must be sent to the patient for the purpose of making the CT images only to be sent back immediately afterwards in order to a make the necessary adjustments. Furthermore, the method is cannot be used if one or more fiducial marker are not visible in the CT image, for example due to artifacts.

### SUMMARY OF THE INVENTION

An object of the present invention is to provide a method and apparatus for the design and manufacturing of custom patient specific guiding templates, which avoids the problems associated with working solely with medical images and provides an easier means to go to production.

The present invention provides a method for designing a guiding template or a precursor thereof using a computing device having a processor and a memory, the guiding template or the precursor thereof being for implantology or surgery for a patient in need thereof, comprising:
providing a plurality of separate digital scanning images, at least one of the images being of an anatomical part of the patient;
combining the plurality of digital scanning images by means of computer assisted registration; and generating the design of the guiding template or a precursor thereof from the combined image. Constituent parts of the guiding template or the precursor thereof can be a contact element and one or more guiding elements. The generated design can include the contact element and the one or more guiding elements. One or more designs of the contact element can be obtained by digitally manipulating at least one of the digital scanning images and one or more designs of the guiding elements can be obtained by digitally manipulating the remaining digital scanning images.

The contact element can be for support either by a part of the body of the patient or by reference elements fixed to the patient. One or more of the digital scanning images used for designing the contact element can be acquired by means of a surface scan or a topographical measurement of a part of the body of the patient which is to provide support or of reference elements fixed to the body of the patient which are to provide support. The topographical measurement of the part of the body can be done, for example, either directly on the patient or indirectly on a structure containing the geometrical information and providing a surface description of this body part and/or of the reference elements fixed to the body. An example would be a plaster or polymer cast of the jaw of the patient. The reference elements fixed to the body can be implants that are in position prior to scanning.

One or more of the digital scanning images used for designing the guiding elements can comprise a digitized volumetric description of a part of the body identified for treatment planning. Such volumetric description can be generated by methods which record volumetric structure of a patient such as X-ray or MRI scans.

Computer assisted registration of one or more digital scanning images can be done by means of volume to volume registration, or a surface to surface registration, or by point registration. A surface for the surface to surface registration can be obtained by converting a volumetric description to a surface image or from a surface scan image. Surface to surface matching can be performed on all or on selected parts of surface images.

The computer assisted registration of one or more digital scanning images can alternatively be done by means of 3-point or multiple point registration, in which these points are determined either by fiducial markers or by anatomical landmarks visible in the images. The multipoint registration can use at least 1000 points, e.g. 10,000 points. By use of many points the registration errors can be reduced.

The digital manipulations on the scanning images used to create the design of the contact element can apply offset operations and boolean operations to provide digitally a thickness to the surface description of part of the anatomy. The digital manipulations on scanning images to design the guiding elements can comprise a computer assisted planning of the implantology or surgery. The computer assisted planning of the intervention can result in information about the position, shape, the size, the orientation and the dimensions of the guiding elements.

Constituent parts of the guiding template or the precursor thereof can be integrated by means of Boolean operations and are output as a digital description of the guiding template.

The guiding template can be manufactured directly based on the output design or the guiding template can be manufactured indirectly using the precursor, e.g. using Computer aided manufacturing techniques and equipment. For example, the design of the guiding template or the precursor thereof can be processed to manufacture the guiding template by rapid prototyping techniques.

The present invention also includes an apparatus for designing a guiding template or a precursor thereof for implantology or reconstructive surgery, the apparatus comprising:
a computing device having a processor and a memory,
means for receiving a plurality of separate digital scanning images, at least one of the images being of an anatomical part of the patient;
means for combining the plurality of digital scanning images by computer assisted registration; and means for generating the design of the guiding template or a precursor thereof from the combined image, and
means for outputting a digital description of the design of the guiding template. Constituent parts of the guiding template or the precursor thereof can be a contact element and one or more guiding elements. The generated design can include the contact element and the one or more guiding elements. The apparatus can include means for digitally manipulating at least one of the digital scanning images to obtain one or more designs of the contact element. The apparatus can also include means for digitally manipulating the remaining digital scanning images to obtain one or more designs of the guiding elements.

The means for computer assisted registration of one or more digital scanning images can include means for volume to volume registration, or means for a surface to surface registration, or means for point registration. The means for the surface to surface registration can include means for converting a volumetric description to a surface image. The apparatus can include means for surface to surface matching on all or on selected parts of surface images.

The means for computer assisted registration of one or more digital scanning images can alternatively include means for 3-point or multiple point registration, in which these points are determined either by fiducial markers or by anatomical landmarks visible in the images. The multipoint registration can use at least 1000 points, e.g. 10,000 points.

The means for digital manipulations of the scanning images to create the design of the contact element can include means to apply offset operations and boolean operations to provide digitally a thickness to the surface description of part of the anatomy. The means for digital manipulations of scanning images to design the guiding elements can comprise means for computer assisted planning of the implantology or surgery. The means for computer assisted planning of the intervention can output information about the position, shape, the size, the orientation and the dimensions of the guiding elements.

Constituent parts of the guiding template or the precursor thereof can be integrated by means for Boolean operations which can output a digital description of the guiding template.

The present invention provides a computer program product comprising code segments, the code segments comprising, when executed on a computing device:
means for receiving a plurality of separate digital scanning images, at least one of the images being of an anatomical part of the patient;
means for combining the plurality of digital scanning images by means of computer assisted registration; and means for generating the design of the guiding template or a precursor thereof from the combined image. The computer program product can comprise means to carry out any of the methods of the present invention. The present invention also includes a machine readable storage medium storing the computer program product.

Advantages of the method and apparatus according to the present invention include: a precise fit of the guiding template even in cases with a lot of artifacts in the images, the fact that after the scanning procedure, parts of corresponding surfaces of the at least two images can be chosen to perform the registration which results in a better precision of registration of the two images and this can result in a better and more stable fit of the template during surgery, especially during drilling using the drill guides.

Other details and advantages of the invention will emerge from the description that follows of a number of embodiments of the method and apparatus according to the invention. This description is given merely as an example and does not limit the scope of the protection claimed. The reference numbers below relate to the appended figures.

### BRIEF DESCRIPTION OF THE FIGURES

Figures 1 and 2 show a volumetric image having artifacts generated by metal fillings in teeth.
Figure 3 is a diagrammatic view of a guiding template.
Figure 4 is view of a plaster cast fabricated by the dental lab based on an impression of the patient.
Figure 5 shows a triangulated digital surface description of a patient's jaw based on surface measurements made on a plaster cast.
Figure 6 shows the registration of a digital surface description with a volumetric description of the same jaw. Two proposed dental implants are also shown.
Figure 7 shows how the contact element and the guiding elements are combined to obtain a guiding template.
Figure 8 is a schematic representation of a computer based apparatus for carrying out the methods according to embodiments of the present invention, including optional computer aided manufacturing.

### DETAILED DESCRIPTION OF THE ILLUSTRATIVE EMBODIMENTS

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes.

The terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

It is to be noticed that the term "comprising", used in the claims, should not be interpreted as being restricted to the means listed thereafter; it does not exclude other elements or steps.. Thus, the scope of the expression "a device comprising means A and B" should not be limited to devices consisting only of components A and B. It means that with respect to the present invention, the only relevant components of the device are A and B.

In the following and in the attached claims reference will be made to a "patient". It should be understood that this term patient should be construed broadly to include not only humans but also animals, especially mammals in need of surgery.

Methods for the production of bone-supported drill guides based on CT-images are already used in dental implantology for optimizing the treatment of patients with edentulous or partially edentulous jaw. In some cases however - for example when only one or a couple of teeth are missing - there is insufficient space between the still remaining teeth to allow the use of a bone supported guide. In a bone supported guide the overlying soft tissue must be moved aside to allow placing of the guide. In accordance an aspect of the present invention this additional operative step can be avoided. In these cases, a guide supported on other features such teeth, gums or even earlier placed implants provides a solution.

Design and manufacturing of this type of guiding template can be done in 3 and 4 phases, respectively:
(i) digitization of the information specific for the patient
(ii) simulation of the intervention in a planning software
(iii) computer assisted design of a precursors of a guide or of the guide itself based on detailed information obtained in phase 1 and on the planning information obtained in phase 2. A precursor of a guide is a template from which the final template can be manufactured, e.g. it is a negative impression of the final guide.
(iv) production of the guides
The present invention provides a method and apparatus for (i) combining a computer planning based on one or more medical images of the patient with geometrical information of the supporting surfaces in digital format (ii) processing these data in order to obtain a digital design of a custom guiding template that (iii) can be manufactured, for example, by means of CAM techniques. In order to be able to combine the detailed information of the supporting geometries mentioned above with the planning information, the digital description of the surfaces must be positioned to the corresponding surfaces present in the medical images identically in a suitable display space (this is known as « registration »). Only when this is the case, can one refer to a single coordinate system and the planning information can be usable directly without the necessity for a coordinate transformation. The accurate repositioning of the digital surface model can be done by means of a so-called registration algorithm. Typically a distance-function will be generated, quantifying the distance between points in the point clouds describing the objects. Next, a transformation is calculated that minimizes this distance function.

The contact element of the guiding template can be designed based on the registered surface description of the support structures. This can be done by software-matically selecting a number of characteristic support features. By assigning these features a thickness, a contact element is obtained that near to perfectly fits the anatomical support structures. The resulting template can be a patient anatomy supported guiding template or a template supported on other objects, e.g. on other pre-inserted implants.

The design of the guiding elements is based on a pre-operative planning. Since contact element and guiding elements are positioned correctly with respect to each other in space, they can be processed directly into an integrated design of the guiding template. The present invention includes methods and apparatus for designing a guiding template or a precursor thereof based on multiple scans and registration of the images generated by these scans. The contact element of the guiding template is designed, based upon at least one of the scans, and the design of the guiding elements is based upon a planning which is done in a scan or scans containing volumetric information. The scan or scans used for designing the contact surface can be a surface scan. The surface information can be obtained by scanning the supporting structures directly or indirectly be scanning an object containing information about the topography of the object such as a plaster model. The information, e.g. relating to guiding elements/contact element, is combined by registration of the images. The registration can be a volume to volume registration, a surface to surface registration, a registration of parts of surfaces or a point to point registration. The guiding templates can be designed so that they will be supported on parts of the body but also on references objects fixed to the body, for example implants placed prior to scanning.

In accordance with an aspect of the present invention digitization of the topography of a part of the anatomy of a patient, e.g. the human or animal jaw - in casu teeth, gums, temporary or already present implants - is done by means of two separate images: e.g. volumetric (e.g. MRI, CT, PET, ultrasound) and surface measurements (e.g. laser holography, laser scanners, stereo photographs, mechanical digitizers, mechanical sensor, etc.). The former are performed on the patient and the latter are performed either on the patient, e.g. intra-orally or on a structure which is an impression of the patient's anatomy, e.g. a dental-technical structure (a plaster cast, a diagnostic (wax) teeth setup, a scan prosthesis, etc.) manufactured by a dental technician specifically for the patient. This structure is typically based on a physical impression made by the dentist or surgeon. An example of such an impression is given in Fig. 4. The result of the surface scan is a digital image which can be displayed as shown in Fig. 5. The surface image can be obtained by any suitable scanning method of the cast as shown in Fig. 4 or by a direct scanning of the relevant part of the patient's anatomy. A cast such as shown in Fig. 4 may also be captured by means of a high definition volumetric scanning method. Due to the fact that the cast is inanimate, volumetric scanning parameters and times can be used that would not be possible for a patient. By this means the resolution of the volumetric image can be improved compared with patient scans. From this high definition scan a surface rendering can be derived, for example using the marching cubes algorithm (Lorensen, W.E. and Cline, H.E., "Marching Cubes: a high resolution 3D surface reconstruction algorithm," Computer Graphics, Vol. 21, No. 4, pp 163-169 (Proc. of SIGGRAPH), 1987.) As the cast does not need to contain metal objects, distortions and artifacts can be avoided. Also, with only one density of material, thresholding errors are reduced. However, direct surface scanning methods are preferred over surface scans generated from volumetric images.

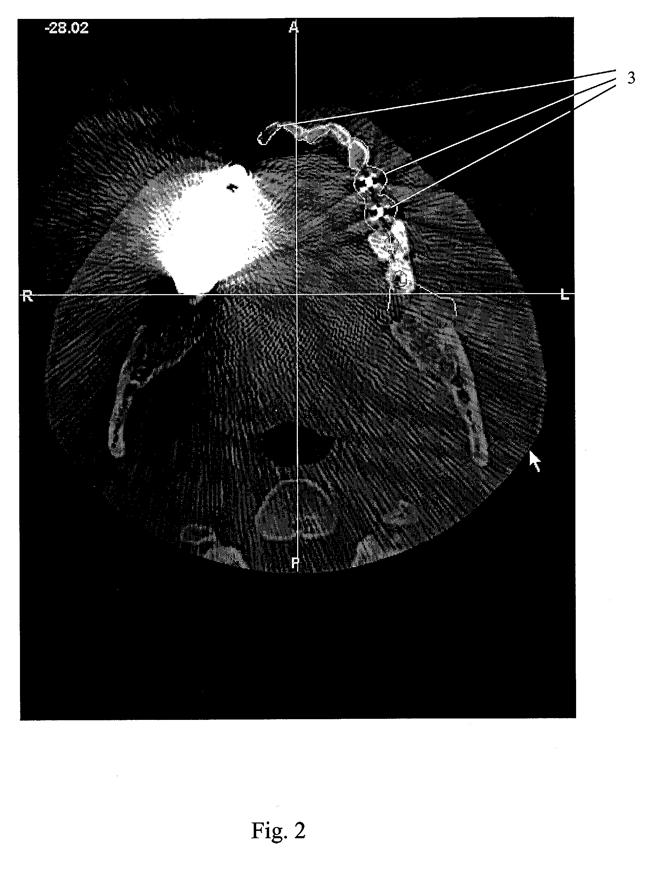

The reason for using surface measurements or a surface derived rendering based on high resolution volumetric scans is fourfold: (i) the outline/contours of the teeth in volumetric medical images taken directly from living patients are often distorted due to artifacts (see Fig. 1), (ii) the outlines are too jagged and rough due to suboptimal resolution and (iii) image detail is very threshold dependent as the patient is composed of materials with varying densities. The volumetric image may even lack certain features in areas close to the artifacts. This can be seen when comparing Figs. 1 and 2. The tooth 3 whose perimeter is indicated in Fig. 2 is missing or is improperly represented in Fig. 1 Finally, (iv) any surface description derived from volumetric information of patients, e.g. a medical image such as MRI, will be influenced by a variety of difficulties, e.g. the interpolation technique used to interpolate between consecutive slices having a finite thickness. This can reduce resolution and/or accuracy of the image. For specific scan techniques there are further factors such as (for MRI) phase mismapping, aliasing, chemical shift, chemical misregistration, motion artifact, magnetic susceptibility artifact, zipper artifact, shading artifact, cross-talk and cross excitation - see the book MRI in practice, C. Westerbook and C. Kaut, 2^{nd} ed., Blackwell Science, 1998, especially chapter 7. To eliminate some artifacts from a volumetric image derived from a patient, the image may be segmented, i.e. any distorted area is removed from the image. However, once removed the information is lost.

Using surface measurements, the entire topography can be captured in a precise, automated and cost-effective manner without the above-mentioned problems. The result of the digitization is a point cloud or triangulated description of the topography of the teeth/gums or another part of the body. The resolution or accuracy of this surface scan is preferably at least less than 100 micron along any axis.

The digital design of the guiding templates is based on the planned implant positions relative to the coordinate system present in the volumetric medical images and on the detailed topographical description of the teeth/gums/implant fixtures in the surface image. In order to combine these input images, a registration is performed, aligning the detailed topographical information with the corresponding segmentation in the medical images. Registration may be defined as a process of finding a set of transformation operations between two or more image data sets such that an overlap between the images generated by these sets in a certain common space minimizes a certain optimization criterion. Generally, the optimization criterion is a criterion related to a distance metric derived from a plurality of distances between the two images or surfaces when overlapped. The plurality of distances is preferably distances between greater than or equal to 3 points, more preferably greater than or equal to 1000 points and most preferably greater than or equal to 10,000 points. This registration can be performed by a number of methods. Firstly, the cloud of points which represents the surface image (or selected parts of this surface) is registered with the cloud of points which represents the surface extracted from the volumetric image (or parts of it) - suitable methods are described in "Closed form solution of avsolute orientation using unit quaternions", by B. K. P Horn, J. Opt. Soc. Amer. A vol. 4, no. 4, pp. 629-642, April 1987 and/or "A method of registration of 3D shapes", by P. J. Besl, N. McKay, IEEE Transactions on pattern analysis and machine intelligence, vol. 14, no. 2, pp. 239-255, Feb. 1992. This registration may include statistical methods to improve the registration, e.g. where points in the volumetric image lie more than a certain tolerance from the position of the surface image, e.g. more than three times the standard deviation of the differences between the two images, these points may be discarded and a new registration performed. Secondly, although, the above method is preferred, the registration may be made using stereotactic (fiducial) reference points (i.e. point to point registration). For example, where implants have already been placed, details of these implants may be used for registration. The existing implants will appear in both a volumetric image of the patient and the cast of the patient's jaw. In this case a subset of points of the volumetric image and a sub-set of points for the scanned image are used for registration. For information on volume to volume registration see the book by Suetens mentioned above.

Where artifacts have been segmented out of a volumetric image, the surface data corresponding to the removed section is replaced by the surface image. Preferably, for the purpose of accurate registration, any area segmented in the medical images that is used for registration should contain as few as possible artifacts.

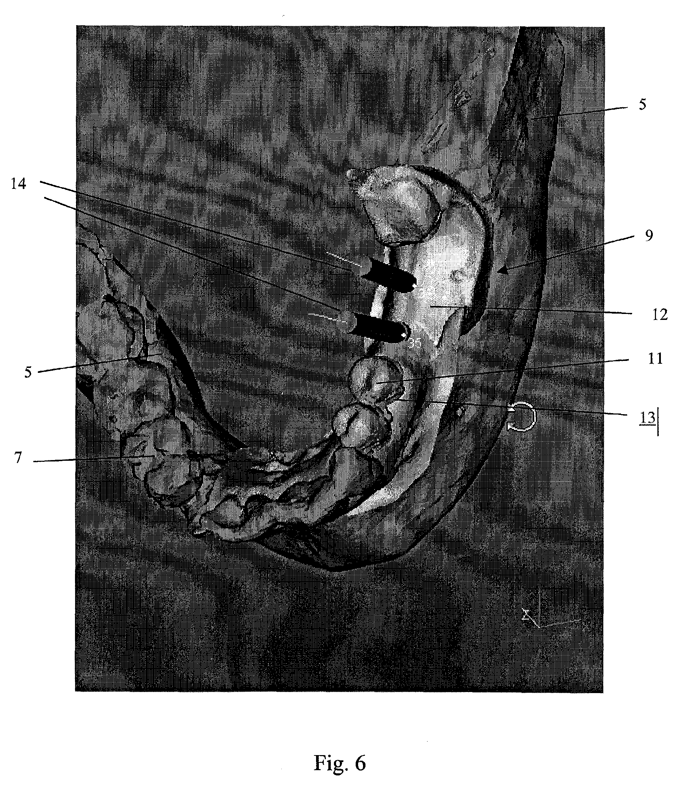

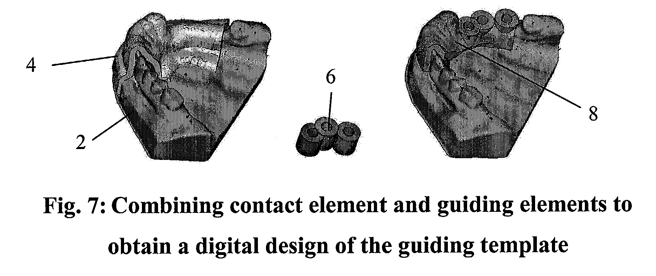

Using software tools such as boolean operations, scaling, offsets a. o., the digital information about support structures and implant planning is merged to obtain a displayable computer model of the tooth supported guiding template. An example of such a merged image is shown in Fig. 6. Item 5 is a surface representation of the teeth and soft tissue generated from a volumetric image, e.g. CT. Teeth as shown at position 7 are irregular and have a poor correlation with the actual teeth. This part of the image is not suitable as a basis for making a template or a precursor of a template. However, this region of the image is not relevant for the surgical intervention. At least one surface image 9 generated by a surface scan is registered with the volumetric image 5. The definition of the teeth at this position, e.g. 11, as well as of the soft tissue 12 are much superior to the same teeth as would be generated from the volumetric image 5. Further, where artifacts are present in the volumetric image 5 these can be removed, e.g. by manual manipulation of the image 5, before merging of the two images 5, 9. By coloring the images 5, 9 differently, the two images can be distinguished even when merged. For example, at 13 a portion of the volumetric image penetrates above the surface image 5. As the surface image 5 generated by a surface scan is more accurate than a surface image derived from a volumetric image 9 of the patient's jaw, it can be assumed that such excursions or incursions are errors. In accordance with an aspect of the present invention, means are provided in the digital visualization software to select those parts of the merged images 5, 9 that are to be used for the design of the template or its precursor, e.g. to exclude regions which are unsuitable. Such means may include a means to crop the unwanted parts of the image such as at 13. Also shown are two proposed implants 14 that are superimposed on the merged images. The implants may be generated by the graphics software program used to display the images 5, 9, may be recalled from a library, etc. and may be manipulated (e.g. rotated, translated, scaled, copied, brought to foreground or background, brought above or below another image, generated from a boolean operation between two images, etc.) in accordance with normal operations for 3-D graphics or CAD software. The merged images can be used for both planning of the surgical intervention, e.g. using details of important anatomical structures from the volumetric image 5 such as bone, blood vessels or nerves, as well as design of the template, e.g. using surface information from the surface image to allow accurate location of the template. A completed guide 8 will typically consist of a number of guiding elements 6 corresponding to the orientation of the planned implants (with or without heights to provide depth control) and a contact element 4 that ensures the unique and stable positioning of the guide on the teeth - see Fig. 7.

According to an aspect of the present invention, the contact element is designed based on the topography of the support structures derived from patient information.
In accordance with an embodiment of the present invention the production of the teeth supported guiding templates, starting from the computer design 8 described above can be done by means of rapid prototyping techniques. The digital template design 8 is translated into layered sections that will build up the physical template, (cf. WO 9729901 Method and device for making a three-dimensional object from a hardenable liquid medium, Materialise N.V., Leuven, Belgium)

Stereolithography allows manufacture of very thin layers; the materials used can be non-toxic (USP class VI) and have according to the prior art been used for manufacturing bone supported guiding templates (cf. SurgiGuides^{TM}, Materialise N.V., Leuven, Belgium) without the novel and inventive features of the present invention.

In a variant of the above method, the teeth supported guiding template or a precursor thereof such as a negative impression thereof is manufactured in wax by means of 3D plotting according to the inkjet principle. Afterwards, the wax template can be used to fabricate an identical template in another material (including metals), e.g. by casting.

In the proposed way of working according to the present invention it suffices to send a computer planning and a (digital or physical) model of support structures once to enable the design and manufacturing of a guiding template.

Possible applications of the invention will be elucidated further by means of a number of examples.

### Example 1: Tooth supported guiding template in combination with local bone support for placement of distal implants.

For placement of distal implants, there are cases where sufficient teeth are present in the frontal region but no molars to provide additional stability in the distal area. Using a tooth supported guide is possible though it would mean that the guide would act as a cantilever.

In these cases one can opt to provide local, additional support in the distal area on the jawbone. This will ensure improved stability of the template and reduce stresses in the template during its use.

The information about the geometry of the jaw bone, necessary for creating a contact element fitting this bone originates from the medical images used to perform the pre-operative planning. The local bone supported contact element is integrated into the design of the guiding template during the design phase.

### Example 2: Tooth supported guiding template in combination with local mucosal support for placement of distal implants.

In a variant of the above application, the additional local contact element is designed to be supported by the gums. This has the advantage of allowing a less invasive approach : a local incision into or local removal (punching/scorching) of the gingiva at the entry point of the implant suffices. This as opposed to opening up the gingiva entirely to free the underlying bony structure.

In comparison to cases with additional local bone support, the contact element will be designed based on the information contained in the detailed surface measurements rather than that in the medical images.

### Example 3: Implant supported guiding template

For some patients it may be necessary to extend an existing prosthesis. Most of the time there will be insufficient teeth left to warrant the use of a tooth supported guide. A guiding template supported by previously placed or temporary implants can offer a solution.

Since the topography of the jaw in these cases will generally contain very few characteristic features usable for registration purposes, radio-opaque copings must be placed on the implants. Using medical imaging techniques a first scan of the patient is taken. Detailed topographical information is obtained by surface measurements on a model of the jaw with copings. Registration is executed using the copings. The further design and production of the guiding template is done conform the above mentioned methods.

### Example 4: Skin supported guiding template for the placement of implants used for fixing a nose/ear prosthesis

Important for the placement of implants intended for fixation of ear/nose prostheses in reconstructive surgery is an adequate pre-operative planning and its subsequent transfer to the operating theatre. A common risk lies in injuring important nerve bundles and/or blood vessels. A surgical guiding template, fabricated corresponding to the above-mentioned method can prove to be a good solution.

Planning of the implant positions is done using medical images, which give volumetric information, thus enabling the surgeon to identify good quality bone. Typically a CT scan will be used. A support structure on the skin will be used. Soft tissues are difficult to segment in CT images. In addition a relatively large contact element is advisable to assure a stable position of the template despite the fact that the skin is deformable to a certain extent. A large contact element however conflicts with the need to minimize the area exposed to radiation during CT.

By performing an additional topographical scan of the skin (or a model base on an impression) and combining this information with the planning information using registration, a guiding template can be manufactured having a sufficiently large contact element and an accurate fit without above mentioned problems.

### An Implementation of the invention

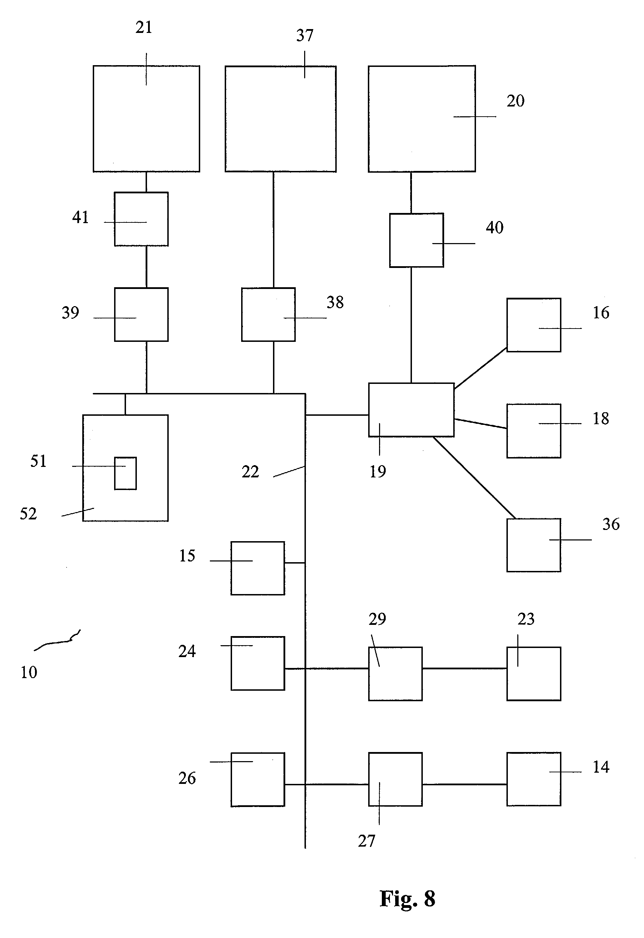

Fig. 8 is a schematic representation of a computing system which can be utilized with the methods and in a system according to the present invention. A computer 10 is depicted which may include a video display terminal 14, a data input means such as a keyboard 16, and a graphic user interface indicating means such as a mouse 18. Computer 10 may be implemented as a general purpose computer, e.g. a UNIX workstation or a personal computer.

Computer 10 includes a Central Processing Unit ("CPU") 15, such as a conventional microprocessor of which a Pentium IV processor supplied by Intel Corp. USA is only an example, and a number of other units interconnected via bus system 22. The bus system 22 may be any suitable bus system - Fig. 8 is only schematic. The computer 10 includes at least one memory. Memory may include any of a variety of data storage devices known to the skilled person such as random-access memory ("RAM"), read-only memory ("ROM"), non-volatile read/write memory such as a hard disc as known to the skilled person. For example, computer 10 may further include random-access memory ("RAM") 24, read-only memory ("ROM") 26, as well as a display adapter 27 for connecting system bus 22 to a video display terminal 14, and an optional input/output (I/O) adapter 29 for connecting peripheral devices (e.g., disk and tape drives 23) to system bus 22. Video display terminal 14 can be the visual output of computer 10, which can be any suitable display device such as a CRT-based video display well-known in the art of computer hardware. However, with a desk-top computer, a portable or a notebook-based computer, video display terminal 14 can be replaced with a LCD-based or a gas plasma-based flat-panel display. Computer 10 further includes user interface adapter 19 for connecting a keyboard 16, mouse 18, optional speaker 36, as well as allowing optional physical value inputs from physical value capture devices such as medical imaging equipment 40 of an external system 20. System 20 may be connected to bus 22 a data network such as the Internet, an Intranet a Local or Wide Area network (LAN or WAN) or a CAN. The medical imaging equipment 40 may be any suitable equipment for capturing volumetric 3-D data of a patient's anatomy such as CT, MRI, PET, ultrasound. This medical imaging equipment may include any device for capturing relevant volumetric image data of a patient. A second patient anatomical data capturing device 41 is provided in a further system 21 for capturing topographical data of a patient's anatomy and may be connected to bus 22 via a communication adapter 39 connecting computer 10 to a data network such as the Internet, an Intranet a Local or Wide Area network (LAN or WAN) or a CAN. Systems 20 and 21 allow transmission of data relating to physical information of a patient to be transmitted over a telecommunications network, e.g. entering volumetric and topographical descriptions of a patient's anatomy at a remote location and transmitting it to a near location, e.g. via the Internet, where a processor carries out a method in accordance with the present invention. The results of the method may be transmitted to a further near or remote location, e.g. a CAD/CAM processing facility to manufacture the template in accordance with the details provided by computer 10.

The present invention also includes within its scope that the relevant volumetric and topographical data are input directly into the computer using the keyboard 16 or from storage devices such as 23.

A CAD/CAM manufacturing unit 37 may also be connected via a communications adapter 38 to bus 22 connecting computer 10 to a data network such as the Internet, an Intranet a Local or Wide Area network (LAN or WAN) or a CAN. The manufacturing unit 37 may receive an output value or template descriptor file directly from computer 10 running a computer program for template design in accordance with the present invention or a value or descriptor file derived from such an output of computer 10. Alternatively, the unit 37 may receive the relevant design data indirectly on a suitable signal storage medium such as a diskette, a replaceable hard disc, an optical storage device such as a CD-ROM or DVD-ROM, a magnetic tape or similar.

Computer 10 also includes a graphical user interface that resides within machine-readable media to direct the operation of computer 10. Any suitable machine-readable media may retain the graphical user interface, such as a random access memory (RAM) 24, a read-only memory (ROM) 26, a magnetic diskette, magnetic tape, or optical disk (the last three being located in disk and tape drives 23). Any suitable operating system and associated graphical user interface (e.g., Microsoft Windows, Linux) may direct CPU 15. In addition, computer 10 includes a control program 51 that resides within computer memory storage 52. Control program 51 contains instructions that when executed on CPU 15 allow the computer 10 to carry out the operations described with respect to any of the methods of the present invention.

Those skilled in the art will appreciate that the hardware represented in Fig. 8 may vary for specific applications. For example, other peripheral devices such as optical disk media, audio adapters, or chip programming devices, such as PAL or EPROM programming devices well-known in the art of computer hardware, and the like may be utilized in addition to or in place of the hardware already described.

In the example depicted in Fig. 8, the computer program product for carrying out the method of the present invention can reside in any suitable memory. However, it is important that while the present invention has been, and will continue to be, that those skilled in the art will appreciate that the mechanisms of the present invention are capable of being distributed as a computer program product in a variety of forms, and that the present invention applies equally regardless of the particular type of signal bearing media used to actually carry out the distribution. Examples of computer readable signal bearing media include: recordable type media such as floppy disks and CD ROMs and transmission type media such as digital and analogue communication links.

Accordingly, the present invention also includes a software product which includes which when executed on a suitable computing device carries out any of the methods of the present invention. In particular, the code includes means for receiving a plurality of separate digital scanning images, at least one of the images being of an anatomical part of the patient; means for combining the plurality of digital scanning images by computer assisted registration; and means for generating the design of the guiding template or a precursor thereof from the combined image. The code may include means for outputting a digital description of the design of the guiding template. Constituent parts of the guiding template or the precursor thereof can be a contact element and one or more guiding elements. The generated design can include the contact element and the one or more guiding elements. The code can include means for digitally manipulating at least one of the digital scanning images to obtain one or more designs of the contact element. The code can also include means for digitally manipulating the remaining digital scanning images to obtain one or more designs of the guiding elements.

The code of for computer assisted registration of one or more digital scanning images can include code for volume to volume registration, or code for a surface to surface registration, or code for point registration. The code for the surface to surface registration can include code for converting a volumetric description to a surface image. The code can include means for surface to surface matching on all or on selected parts of surface images.

The code for computer assisted registration of one or more digital scanning images can alternatively include means for 3-point or multiple point registration, in which these points are determined either by fiducial markers or by anatomical landmarks visible in the images. The multipoint registration can use at least 1000 points, e.g. 10,000 points.

The code including means for digital manipulations of the scanning images to create the design of the contact element can include means to apply offset operations and boolean operations to provide digitally a thickness to the surface description of part of the anatomy. The means for digital manipulations of scanning images to design the guiding elements can comprise means for computer assisted planning of the implantology or surgery. The means for computer assisted planning of the intervention can output information about the position, shape, the size, the orientation and the dimensions of the guiding elements.

Constituent parts of the guiding template or the precursor thereof can be integrated by means for Boolean operations which can output a digital description of the guiding template.

Suitable software can be obtained by programming in a suitable high level language such as C and compiling on a suitable compiler for the target computer processor.

The above description discloses several methods and materials of the present invention. This invention is susceptible to modifications in the methods and materials, as well as alterations in the fabrication methods and equipment. Such modifications will become apparent to those skilled in the art from a consideration of this disclosure or practice of the invention disclosed herein. Consequently, it is not intended that this invention be limited to the specific embodiments disclosed herein, but that it cover all modifications and alternatives coming within the true scope and spirit of the invention as embodied in the attached claims.

## Claims

1. Method for designing a guiding template or a precursor thereof using a computing device having a processor and a memory, the guiding template or the precursor thereof being for implantology or surgery for a patient in need thereof, comprising:
providing a plurality of separate digital scanning images, at least one of the images being of an anatomical part of the patient;
combining the plurality of digital scanning images by means of computer assisted registration; and generating the design of the guiding template or a precursor thereof from the combined image.

2. The method according to claim 1, wherein the constituent parts of the guiding template or the precursor thereof comprise a contact element and one or more guiding elements.

3. Method according to claim 2, wherein one or more designs of the contact element are obtained by digitally manipulating at least one of the digital scanning images and wherein one or more designs of the guiding elements are obtained by digitally manipulating the remaining digital scanning images.

4. Method according to claim 2 or 3, wherein one or more of the digital scanning images used for designing the contact element are acquired by means of a surface scan or a topographical measurement of a part of the body of the patient which is to provide support or of reference elements fixed to the body of the patient which are to provide support.

5. Method according to claim 4, wherein the topographical measurement of the part of the body is done either directly on the patient or indirectly on a structure containing the geometrical information and providing a surface description of this body part and/or of the reference elements fixed to the body.

6. Method according to claim 4 or 5, wherein the reference elements fixed to the body are implants that are in position prior to scanning.

7. Method according to any previous claim, wherein one or more of the digital scanning images used for designing the guiding elements comprise a digitized volumetric description of a part of the body identified for treatment planning.

8. Method according to any previous claim, wherein computer assisted registration of one or more digital scanning images is done by means of one of volume to volume registration or surface to surface registration or 3-point or multiple point registration, in which these points are determined either by fiducial markers or by anatomical landmarks visible in the images.

9. Method according to claim 8, wherein a surface for the surface to surface registration is obtained by converting a volumetric description to a surface image.

10. Method according to claim 8 or 9, wherein surface to surface matching is performed on selected parts of surface images.

11. Method according to any of the claims 4 to 10, wherein the digital manipulations on the scanning images used to create the design of the contact element apply offset operations and boolean operations to provide digitally a thickness to the surface description of part of the anatomy.

12. Method according to any of claims 3 to 11, wherein the digital manipulations on scanning images to design the guiding elements comprise a computer assisted planning of the implantology or surgery.

13. Method according to claim 12, wherein the computer assisted planning of the intervention results in information about the position, shape, the size, the orientation and the dimensions of the guiding elements.

14. Method according to any previous claim, wherein constituent parts of the guiding template or the precursor thereof are integrated by means of Boolean operations and are output as a digital description of the guiding template.

15. Method according to any previous claim, further comprising manufacturing the guiding template directly based on the output design or manufacturing the guiding template indirectly using the precursor.

16. Method according to claim 15, wherein the design of the guiding template or the precursor thereof is processed to manufacture the guiding template by means of rapid prototyping techniques.

17. An apparatus for designing a guiding template or a precursor thereof for implantology or reconstructive surgery, the apparatus comprising:
a computing device having a processor and a memory,
means for receiving a plurality of separate digital scanning images, at least one of the images being of an anatomical part of the patient;
means for combining the plurality of digital scanning images by computer assisted registration; and means for generating the design of the guiding template or a precursor thereof from the combined image, and means for outputting a digital description of the design of the guiding template.

18. A computer program product comprising code segments, the code segments comprising, when executed on a computing device:
means for receiving a plurality of separate digital scanning images, at least one of the images being of an anatomical part of the patient;
means for combining the plurality of digital scanning images by means of computer assisted registration; and means for generating the design of the guiding template or a precursor thereof from the combined image.

19. A machine readable storage medium storing the computer program product of claim 18.
